# FASCICULE DE BREVET EUROPEEN

(11) **EP 2 443 227 B1**
(45) Date de publication et mention de la délivrance du brevet: **04.01.2017**
(21) Numéro de dépôt: 10734774.2
(22) Date de dépôt: 17.06.2010
(51) Int. Cl.: C12N 1/04, C12N 1/18, A21D 8/04

(54) **LEVURE, PROCEDE DE PREPARATION, COMPOSITION ET UTILISATIONS**
HEFE, HERSTELLUNGSVERFAHREN, ZUSAMMENSETZUNG UND VERWENDUNG
YEAST, PREPARATION METHOD, COMPOSITION AND USES THEREOF

(30) Priorité: 19.06.2009 FR 0902985
(43) Date de publication de la demande: 25.04.2012
(73) Titulaire: Lesaffre et Compagnie, 75001 Paris (FR)
(72) Inventeur: HIOLLE, Amélie, F-59144 Anfroipret (FR); LENOIR, Christian, F-59520 Marquette Lez Lille (FR); BARTOLUCCI, Jean-Charles, F-59350 Saint-André (FR)
(74) Mandataire: Cabinet Plasseraud
(86) Numéro de dépôt international: PCT/FR2010/000451
(87) Numéro de publication internationale: WO 2010/146260

(56) Documents cités:
- EP-A1- 0 636 692
- CH-A- 332 457
- DE-A1- 19 900 446
- FR-A- 1 312 694
- FR-A2- 2 197 977
- FR-A5- 2 148 727

## Description

L'invention se rapporte à un procédé de préparation d'une levure, à une levure obtenue selon le procédé, à une composition comprenant la levure, à une installation destinée à mettre en oeuvre le procédé de préparation et à différentes utilisations de la levure.

Il est déjà connu du document WO 81/01415 un procédé de préparation de microorganismes secs, actifs, tels que de la levure de boulangerie, ayant un taux de matière sèche compris entre 90 et 98% en poids. Cette levure sèche est obtenue par le séchage d'une masse de levure sur un lit fluidisé, puis éventuellement dans un séchoir à tambour. La dimension moyenne des particules de levure obtenues à l'issue de ce procédé est comprise entre environ 0,4 et 1,5mm.

Mais l'utilisation d'un lit fluidisé dans l'étape de séchage ne permet pas d'obtenir des particules de levure présentant une surface lisse. Elles restent par conséquent poreuses et, présentent donc une moins bonne stabilité dans le temps lorsqu'elles sont mises au contact de l'air.

Le document JP 55162928 décrit par ailleurs un procédé de séchage de levure de boulangerie. Ce procédé consiste à faire sécher la crème de levure en la faisant passer, rapidement et en continu, au travers d'un échangeur de chaleur puis dans un séchoir à tambour de manière à obtenir une levure ayant un taux de matière sèche d'au moins 95% en poids.

Il est encore connu du document JP 05097687 la préparation de levure sèche, sous forme de granule, par un séchage utilisant un ou deux séchoirs à tambours.

Mais de telles techniques de séchage ne permettent pas d'obtenir des particules de levure sèche présentant un pouvoir ferment élevé pendant plusieurs mois tout en ayant une faible reprise d'humidité.

En outre, la levure est connue de manière générale pour être particulièrement sensible aux variations de température et d'hygrométrie liées aux conditions de conservation, ainsi qu' à la présence d'oxygène.

Aussi il subsiste le besoin de pouvoir disposer d'un procédé de fabrication de levure sèche ayant une mise en oeuvre simple et rapide, ledit procédé permettant le lissage de la surface des particules de levure garantissant ainsi une meilleure stabilité de la levure dans le temps quant au maintien de son pouvoir ferment et sa faible vitesse de reprise d'humidité.

L'invention a donc pour objet un procédé de fabrication de la levure sèche se présentant sous forme de sphérules entourées d'une couche protectrice périphérique essentiellement constituée de cellules mortes. Ce procédé se caractérise en ce qu'il consiste à effectuer une déshydratation d'un volume de crème de levure comprenant une saumure à base de chlorure de sodium, une granulation de la levure déshydratée pour l'extruder, puis un séchage de la levure extrudée de manière à obtenir une levure sèche ayant un taux de matière sèche supérieur ou égal à 92% en poids, le séchage étant effectué en discontinu et en l'absence d'additif chimique, et consistant lors d'une première étape, effectuée dans un tambour rotatif, à faire passer un courant d'air chaud dans le volume de levure, puis lors d'une seconde étape, effectuée dans une tour de séchage, à envoyer de l'air chaud sous la partie inférieure du volume de levure.

Ce procédé présente l'avantage de fournir une levure qui reste peu sensible aux variations de température et d'hygrométrie, à la présence d'oxygène, qui présente un pouvoir ferment élevé et une vitesse lente à la reprise en humidité, qui reste stable pendant plusieurs mois, voire un an, au contact de l'air lorsqu'elle est mélangée avec des ingrédients alimentaires, tels que la farine ayant par exemple de 7 à 15% d'humidité, et qui se disperse correctement lorsqu'elle est incorporée dans la pâte.

Un autre objet de l'invention est une levure sèche obtenue selon le procédé décrit précédemment, qui se caractérise en ce qu'elle présente un diamètre moyen compris entre environ 0,4 et 0,7 mm, en ce qu'elle ne comprend pas d'additif chimique, et en ce qu'elle présente une durée de reprise d'humidité comprise entre environ 1 et 2 heures.

Par durée de reprise en humidité, on entend le temps mis par la levure pour atteindre l'humidité de son environnement qui peut être, par exemple un mixe à base de farine.

La levure selon l'invention peut présenter un pouvoir ferment supérieur ou égal à environ 70ml de dégagement gazeux pendant une durée d'au moins six mois à une température comprise entre environ 15° et 25°C, lorsqu'elle est mélangée à une farine ayant au moins environ 13% d'humidité.

De préférence, la levure selon l'invention présente un diamètre moyen compris entre environ 0,4 et 0,7mm, et de préférence d'environ 0,55mm.

La levure selon l'invention présente l'avantage d'avoir une matière sèche homogène, de se disperser facilement et rapidement lorsqu'elle est incorporée à un mélange comprenant de la farine et des ingrédients du fait notamment de sa petite dimension.

La présence d'une couche mince périphérique, protectrice, essentiellement constituée de cellules mortes, faisant barrière à l'oxygène, permet à la levure de cumuler à la fois une faible porosité limitant ainsi sa vitesse de reprise d'humidité par rapport à des levures sèches dépourvues de telle couche protectrice, un bon pouvoir ferment, et une meilleure stabilité dans le temps en comparaison à d'autres levures sèches, notamment de son pouvoir ferment.

La levure selon l'invention présente l'avantage d'avoir une vitesse de reprise d'humidité située entre celle des levures sèches actives connues (dites levures actives) ayant un diamètre supérieur et une couche mince protectrice de cellules mortes, et, celle des levures sèches également connues (dites levures instantanées) n'ayant aucune couche protectrice. Ainsi, la reprise d'humidité de la levure selon l'invention est donc plus rapide que celle des levures sèches actives ayant une couche protectrice, mais plus lente que celle des levures sèches instantanées.

Cette couche protectrice permet également de protéger la levure contre des variations de température lors de sa conservation, susceptible d'altérer son pouvoir ferment et/ou sa viabilité.

Enfin, un autre avantage de la levure selon l'invention est que lorsqu'on la mélange intimement à des ingrédients alimentaires, il ne se produit pas de phénomène de décantation ou d'agglomération au cours du temps. En effet, elle se disperse correctement, notamment dans des aliments à base de farine.

Ceci est dû au fait qu'elle présente une petite taille et qu'elle est entourée d'une couche protectrice périphérique.

Un troisième objet de l'invention est une composition comprenant de la levure telle que mentionnée ci-dessus, au moins un ingrédient choisi parmi le gluten, le sel, les améliorants, les levains, différentes farines de blé ou de seigle, et leur mélange, chaque ingrédient ayant individuellement ou en mélange un taux d'humidité compris entre environ 1 et 16% en poids.

Un quatrième objet de l'invention est une composition comprenant de la levure telle que décrite précédemment, qui se présente sous forme de gélule et/ou de stick et qui ne comprend pas d'excipient.

Un autre objet de l'invention est une utilisation de la levure dans une composition destinée à préparer une pâte boulangère sucrée ou non.

La levure selon l'invention peut être de type osmotolérante ou non, c'est-à-dire pouvoir conserver un pouvoir ferment intéressant lorsqu'elle est incorporée à une pâte sucrée.

Enfin, les derniers objets de l'invention sont des utilisations de la levure, telle que définie précédemment, dans une composition alimentaire pour animal, ou encore, en brasserie et/ou en oenologie.

La déshydratation d'un volume de crème de levure est de préférence effectuée sous vide d'air. Cette étape de déshydratation permet d'obtenir une pâte de levure ayant environ de 30% à 35% en poids de matière sèche à partir d'une crème de levure ayant au départ environ 16% en poids de matière sèche.

A l'issue de l'étape de déshydratation, la levure sous forme d'une pâte est de préférence ensuite extrudée par une étape de granulation qui définit la granulométrie finale de la levure.

Le séchage est effectué en discontinu, et consiste lors d'une première étape, effectuée dans un tambour rotatif, à faire passer un courant d'air chaud dans le volume de levure extrudée, puis lors d'une seconde étape, effectuée dans une tour de séchage, à envoyer de l'air chaud sous la partie inférieure du volume de levure partiellement séché.

La première étape dure de préférence environ 6 heures, et la seconde environ 2 heures.

Lors de la première étape, le courant d'air chaud a de préférence une température comprise entre environ 35° et 38°C, et un débit compris entre environ 20 000 et 30 000m³/h. A l'issue de cette première étape de séchage, le taux de matière sèche de la levure est de préférence d'environ 90% en poids.

Lors de la seconde étape de séchage, le courant d'air chaud a de préférence une température comprise entre environ 41° et 60°C, et un débit compris entre environ 2700m³/h et 4600m³/h selon le tonnage de levure sèche. A la sortie de cette seconde étape de séchage, la levure, qui se présente sous forme de particules plutôt sphériques, a un taux de matière sèche de préférence compris entre environ 92% et 95% en poids.

Contrairement aux techniques connues jusqu'ici, le procédé de préparation selon l'invention n'utilise aucun additif lors du séchage.

La levure issue de ce procédé de préparation peut être incorporée dans une composition alimentaire, destinée à la fabrication d'une pâte boulangère sucrée ou non, en une quantité comprise entre environ 0,40 et 99,5% en poids par rapport au poids total de la composition.

L'invention va maintenant être décrite de manière plus détaillée, à l'aide des figures qui suivent et qui sont données uniquement à titre d'illustration, ainsi que des exemples de composition alimentaire comprenant la levure selon l'invention.
La figure 1 représente l'évolution du pourcentage de matière sèche d'une levure selon l'invention en fonction du temps, lorsqu'elle incorporée dans une farine non étuvée, à une température d'environ 20°C, comparée à celle des levures de l'état de la technique.
La figure 2 représente le temps de dispersibilité de la levure dans la pâte en fonction du temps de reprise en humidité en mixe pour atteindre 89% de matière sèche pour des levures de l'invention et des levures de l'état de la technique.
La figure 3 représente une installation de référence générale 1 décrite ici et destinée à mettre en oeuvre le procédé de préparation de levure.

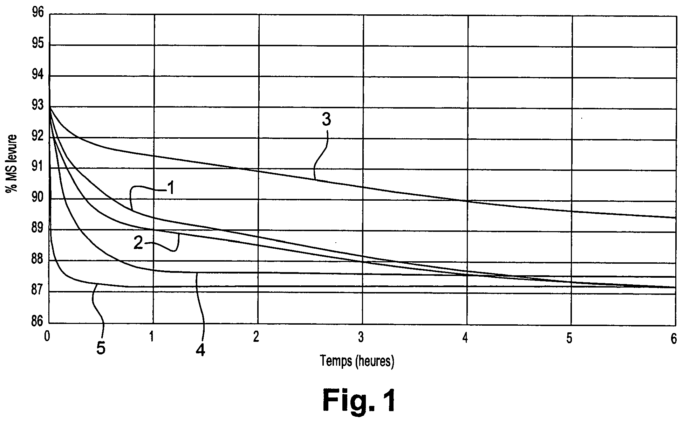

L'évolution du pourcentage de matière sèche de levure sèche en fonction du temps, traduisant sa reprise en humidité (figure 1) est obtenue en appliquant le mode opératoire suivant qui utilise un mélange intime de farine et de levure.

Dans un pot, on place la moitié de la quantité totale de farine. On y ajoute la levure sèche que l'on recouvre ensuite de l'autre moitié de farine. On mélange l'ensemble, on referme le pot et on place ce dernier dans une ambiance de température contrôlée.

Le temps de conservation est décompté à partir de l'instant où la levure est mise en contact avec la farine.

Lorsque le temps de conservation est écoulé, le pot est ouvert, et son contenu est versé sur un tamis de 250 microns d'un tamiseur automatique (de type Bioblock AS200 control g).

A l'issue du tamisage, la levure présente dans le refus du tamis est prélevée, et son pourcentage de matière sèche est déterminé par le calcul mathématique suivant :
poids mesuré après une nuit à 105°C rapporté au poids avant étuvage.

Les différents résultats sont rassemblés sur la courbe de la figure 1.

Comme le montre la figure 1, les courbes de référence 1 et 2 montrent l'évolution du pourcentage de matière sèche de la levure sèche selon l'invention (diamètre moyen compris entre 0,5 et 0,6 mm).

La courbe de référence 3 montre l'évolution du pourcentage de matière sèche d'une levure sèche de l'état de la technique (dite levure active) présentant un diamètre moyen plus élevé (environ 1,8 mm) ayant également une couche périphérique protectrice de cellules mortes.

Les courbes de référence 4 et 5 montrent l'évolution du pourcentage de matière sèche d'une levure sèche de l'état de la technique (dite levure instantanée) se présentant sous forme de fines particules et n'ayant aucune couche périphérique protectrice de cellules mortes.

De ces courbes, on peut constater que le comportement de la levure selon l'invention (références 1 et 2) se situe entre celui des deux types de levure de l'état de la technique (références 3, 4 et 5). La levure selon l'invention met entre environ 1 heure 10 (référence 2) et 1 heure 40 (référence 1) pour atteindre un pourcentage de 89% de matière sèche, à 20°C.

Alors que la levure de référence 3 met environ 10 heures pour atteindre le même pourcentage de matière sèche, et les levures de référence 4 et 5 mettent respectivement environ 18 minutes et 1 minute.

Par conséquent, les levures selon l'état de la technique présentent, soit une vitesse de reprise en humidité très lente mais associée à une incapacité à la dispersion dans la pâte sans réhydratation préalable (référence 3 - levure sous forme de sphérule de gros diamètre environ 1,8 mm connues souvent comme de la levure sèche active), soit une vitesse de reprise en humidité très rapide du fait de l'absence de couche protectrice (références 4 et 5 - levures sèches instantanées).

La levure selon l'invention (références 1 et 2) présente l'avantage de combiner une vitesse de reprise en humidité moyenne grâce à la présence de la couche protectrice tout en ayant une dispersibilité correcte dans la pâte du fait de sa petite taille.

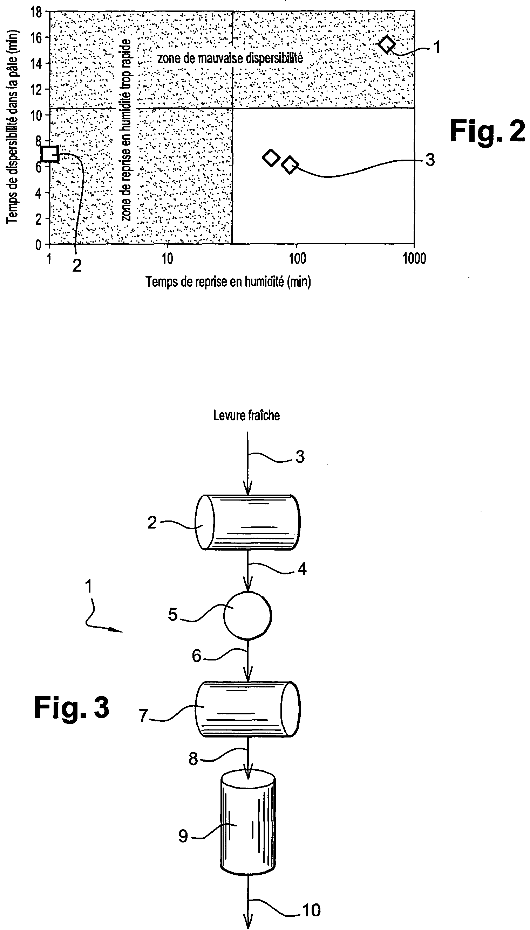

Comme le montre la figure 2, les levures sèches actives 1 ayant un diamètre moyen d'environ 1,8 mm de l'état de la technique présentent un temps de reprise en humidité et un temps de dispersibilité élevés.

Les levures sèches instantanées 2 présentent un temps de reprise en humidité quasi nul pour un temps de dispersibilité correct.

Les levures 3 selon l'invention présentent par contre à la fois un temps de reprise en humidité et un temps de dispersibilité corrects. Elles présentent un bon compromis entre les contraintes de dispersibilité dans la pâte et le temps de reprise en humidité dans un mixe farine.

Les levures de l'état de la techniques 1 et 2 se situent dans des zones de mauvaises dispersibilité et de reprise en humidité trop rapide.

Comme le montre la figure 3, l'installation décrite ici comprend au moins un déshydrateur constitué d'un filtre rotatif 2, alimenté en levure fraîche par une canalisation 3, et relié par une canalisation 4 à un granulateur 5 lui-même relié par une canalisation 6 à un séchoir à tambour 7. Le séchoir 7 est relié par une canalisation 8 à une tour 9 de séchage. Cette dernière est reliée à une canalisation de sortie 10 de la levure sèche.

Le filtre rotatif 2 comprend sur sa surface extérieure une couche de fécule, et tourne à une vitesse d'environ 18 tour /min. La surface du filtre 2 est régulièrement recouverte de fécule.

Le granulateur 5 est constitué d'un premier cylindre avec des bras mélangeurs brassant la levure deshydratée et la poussant vers un second cylindre au bout duquel se trouve une grille ou une tôle perforée pouvant avoir des ouvertures comprises entre 0,5 et 0,8mm en fonction de la granulométrie recherchée pour les particules de levure.

Le séchoir à tambour 7 est constitué d'un cylindre rotatif muni de pâles destinées à brasser la levure, à l'intérieur duquel passe un courant d'air chaud. Sa vitesse de rotation est d'environ 1 tour/min.

La tour 9 de séchage est constituée d'un cylindre dans lequel se trouve une tôle perforée. La levure est disposée sur la tôle et de l'air chaud est envoyée par le dessous permettant ainsi une turbulence de la levure et son séchage final.

L'installation fonctionne de la manière suivante.

Un mélange constitué de levure fraîche à 16% en poids de matière sèche et de saumure comprenant du chlorure de sodium jusqu'à l'obtention d'une conductivité du mélange comprise entre environ 15000 µs et 20000 µS est envoyé, à l'aide de la canalisation 2a, sur le filtre rotatif 2 dont la surface est recouverte au préalable d'une couche de fécule.

A la sortie du filtre rotatif, la levure est déshydratée, et a un taux de matière sèche d'environ 30% à 35% en poids.

La levure déshydratée passe ensuite dans le granulateur 5 pour y être extrudée selon une granulométrie choisie.

La levure extrudée est ensuite dirigée, grâce à la canalisation 6, vers le séchoir à tambour 7 dans lequel elle reste environ 6 heures. L'air chaud qui passe à l'intérieur du séchoir 7 ne subit aucun traitement préalable. La température dans le séchoir 7 augmente lentement et progressivement pour passer d'environ 35° à environ 38°C pendant les 6 heures de séchage.

A la sortie du séchoir 7, la levure est dirigée vers la tour 9 dans laquelle de l'air chaud est envoyé sur le dessus permettant ainsi une turbulence de la levure, et donc son séchage final. La température dans la tour 9 est d'environ 42,5°C pendant toute la période de séchage qui dure environ de 1 à 3 heures.

A la sortie de la tour 9, la levure déshydratée et séchée est ensuite dirigée vers un silo de stockage.

Selon les techniques de boulangerie connues, les compositions alimentaires, illustrant l'invention et faisant l'objet des exemples ci-dessous, sont communément appelés des mixes, des prémixes ou des blends.

On rappelle que les blends sont constitués d'un mélange de levure, de correcteurs de farine et d'améliorants de panification. Les prémixes sont constitués de blends auxquels on ajoute des farines spéciales, du sel, du sucre. Les mixes comprennent des prémixes auxquels on ajoute la farine.

### EXEMPLE 1 Composition alimentaire destinée à préparer un pain type français

| | **Minimum** | **Maximum** |
|---|---|---|
| - Farine de blé Type T45 et/ou T55 et/ou T65 et/ou T80 | 96,92 | 80,66 |
| - Levure 0,6mm | 0,48 | 2,42 |
| - Gluten | 0,09 | 1,61 |
| - Sel fin | 1,45 | 2,01 |
| - Levain sec de blé ou de seigle | 0,97 | 12,09 |
| - Améliorant | 0,09 | 1,21 |
| Total | 100 | 100 |

Les quantités sont données en pourcentage en poids par rapport au poids total de la composition.

L'améliorant comprend différents ingrédients, tels que l'acide ascorbique (de 10 à 100 ppm - de préférence de 20 à 50 ppm), l'alpha amylase fongique (de 0,5 à 15 ppm-de préférence de 3 à 10 ppm), des hémicellulases (de 5 à 150 ppm - de préférence de 15 à 80 ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

Cette composition de mixe, à laquelle on ajoute de l'eau, peut être utilisée dans une machine destinée à fabriquer du pain, ou par pétrissage manuel ou à la spatule.

L'utilisateur de cette composition devra ajouter environ 60 à 62 ml d'eau tiède pour 100 g de composition.

### EXEMPLE 2 Composition alimentaire destinée à préparer un pain brioché

| | **Minimum** | **Maximum** |
|---|---|---|
| - Farine de blé Type T45 et/ou T55 et/ou T65 et/ou T80 | 95,08 | 78,75 |
| - Levure 0,6mm | 0,47 | 2,36 |
| - Gluten | 0,09 | 1,57 |
| - Sel fin | 1,42 | 1,97 |
| - Sucre en poudre | 1,90 | 11,02 |
| - Poudre de lait | 0,95 | 3,15 |
| - Améliorant | 0,09 | 1,18 |
| Total | 100 | 100 |

Les quantités sont données en pourcentage en poids par rapport au poids total de la composition.

L'améliorant comprend différents ingrédients, tels que l'acide ascorbique (de 10 à 100 ppm - de préférence de 20 à 50 ppm), l'alpha amylase fongique (de 0,5 à 15 ppm-de préférence de 3 à 10 ppm), des hémicellulases (de 5 à 150 ppm - de préférence de 15 à 80 ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

Cette composition de mixe, à laquelle on ajoute environ 55 ml d'eau tiède et 8 g de beurre ou margarine pour 100 g de composition alimentaire peut être utilisée dans une machine destinée à fabriquer du pain, ou par pétrissage manuel ou à la spatule.

### Exemple 3 Composition alimentaire destinée à préparer un pain de campagne

| | **Minimum** | **Maximum** |
|---|---|---|
| - Farine de blé type 55 et/ou Type 65 | 85,189 | 69,992 |
| - Farine de seigle T130 | 9,68 | 13,124 |
| - Farine complète de blé | 1,936 | 4,375 |
| - Farine de blé malté | 0,097 | 0,262 |
| - Levain sec de blé et/ou de seigle | 0,968 | 4,374 |
| - Levure 0,6mm | 0,484 | 2,625 |
| - Gluten | 0,097 | 1,749 |
| - Sel fin | 1,452 | 2,187 |
| - Améliorant | 0,097 | 1,312 |
| Total | 100,00 | 100,00 |

Les quantités sont données en pourcentage en poids par rapport au poids total de la composition.

L'améliorant comprend différents ingrédients, tels que l'acide ascorbique (de 10 à 100 ppm - de préférence de 20 à 50 ppm), l'alpha amylase fongique (de 0,5 à 15 ppm - de préférence de 3 à 10ppm), des hémicellulases (de 5 à 150 ppm - de préférence de 15 à 80 ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

Cette composition de mixe, à laquelle on ajoute environ de 62 à 65 ml d'eau tiède pour 100 g de composition alimentaire peut être utilisée dans une machine destinée à fabriquer du pain, ou par pétrissage manuel ou à la spatule.

### Exemple 4 Composition alimentaire destinée à préparer un pain multi graines et céréales

| | **Minimum** | **Maximum** |
|---|---|---|
| - Farine de blé type 45et /ou 55 et/ou Type 65 | 85,84 | 70,68 |
| - Flocons de seigle malté | 5,15 | 8,48 |
| - Graine de tournesol | 1,72 | 4,24 |
| - Graine de lin brun | 1,72 | 3,53 |
| -Eclats de soja | 1,72 | 3,53 |
| - Levure 0,6mm | 0,43 | 2,12 |
| - Gluten | 0,85 | 2,83 |
| - Sel fin | 1,29 | 1,41 |
| - Levain sec de blé ou de seigle | 0,85 | 2,12 |
| - Améliorant | 0,43 | 1,06 |
| Total | 100,00 | 100,00 |

Les quantités sont données en pourcentage en poids par rapport au poids total de la composition.

L'améliorant comprend différents ingrédients, tels que l'acide ascorbique (de 10 à 100 ppm - de préférence de 20 à 50 ppm), l'alpha amylase fongique (de 0,5 à 15 ppm - de préférence de 3 à 10 ppm), des hémicellulases (de 5 à 150 ppm - de préférence de 15 à 80ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

Cette composition de mixe, à laquelle on ajoute environ de 58 à 60 ml d'eau tiède pour 100 g de composition alimentaire peut être utilisée dans une machine destinée à fabriquer du pain, ou par pétrissage manuel ou à la spatule.

### Exemple 5 Composition alimentaire destinée à préparer un pain au seigle

| | **Minimum** | **Maximum** |
|---|---|---|
| - Farine de blé type 55 et/ou Type 65 | 76,55 | 44,44 |
| - Farine de seigle T70 et/ T85 et/ou T130 | 19,14 | 44,44 |
| - Gluten | 0,96 | 2,67 |
| - Sel fin | 1,43 | 1,78 |
| - Levure 0,6mm | 0,48 | 2,67 |
| - Levain sec de blé ou de seigle | 0,96 | 2,67 |
| - Améliorant | 0,48 | 1,33 |
| Total | 100,00 | 100,00 |

Les quantités sont données en pourcentage en poids par rapport au poids total de la composition.

L'améliorant comprend différents ingrédients, tels que l'acide ascorbique (de 10 à 100 ppm - de préférence de 20 à 50 ppm), l'alpha amylase fongique (de 0,5 à 15 ppm-de préférence de 3 à 10 ppm), des hémicellulases (de 5 à 150 ppm - de préférence de 15 à 80 ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

Cette composition de mixe, à laquelle on ajoute environ de 56 à 58 ml d'eau tiède pour 100 g de composition alimentaire peut être utilisée dans une machine destinée à fabriquer du pain, ou par pétrissage manuel ou à la spatule.

### Exemple 6 Composition alimentaire destinée à préparer un pain au son

| | **Minimum** | **Maximum** |
|---|---|---|
| - Farine de blé Type 55 et/ou Type 65 | 86,12 | 71,11 |
| - Son de blé | 9,57 | 17,77 |
| - Gluten | 0,96 | 2,67 |
| - Sel fin | 1,43 | 1,78 |
| - Levure 0,6mm | 0,48 | 2,67 |
| - Levain sec de blé ou de seigle | 0,96 | 2,67 |
| - Améliorant | 0,48 | 1,33 |
| Total | 100,00 | 100,00 |

Les quantités sont données en pourcentage en poids par rapport au poids total de la composition.

L'améliorant comprend différents ingrédients, tels que l'acide ascorbique (de 10 à 100 ppm - de préférence de 20 à 50 ppm), l'alpha amylase fongique (de 0,5 à 15 ppm - de préférence de 3 à 10 ppm), des hémicellulases (de 5 à 150 ppm - de préférence de 15 à 80 ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

Cette composition de mixe, à laquelle on ajoute environ de 60 à 62 ml d'eau tiède pour 100 g de composition alimentaire peut être utilisée dans une machine destinée à fabriquer du pain, ou par pétrissage manuel ou à la spatule.

L'exemple 7 qui suit est une composition alimentaire illustrant l'invention, appelée communément prémixe.

### Exemple 7 Composition alimentaire destinée à préparer un pain brioché

| | **Minimum** | **Maximum** |
|---|---|---|
| - Farine de blé Type 45 et/ou T55 et/ou T65 | 82,67 | 10 |
| - Levure 0,6mm | 1,67 | 10 |
| - Gluten | 0,33 | 6,67 |
| - Sel | 5 | 8,33 |
| - Sucre en poudre | 6,67 | 46,67 |
| - Poudre de lait | 3,33 | 13,33 |
| - Améliorant | 0,33 | 5 |
| Total | 100,00 | 100,00 |

Les quantités sont données en pourcentage en poids par rapport au poids total de la composition.

L'améliorant comprend différents ingrédients, tels que l'acide ascorbique (de 10 à 100 ppm - de préférence de 20 à 50 ppm), l'alpha amylase fongique (de 0,5 à 15 ppm - de préférence de 3 à 10 ppm), des hémicellulases (de 5 à 150 ppm - de préférence de 15 à 80 ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

Cette composition de prémixe peut être utilisée pour préparer un pain brioché, en mélangeant 30 g de prémixe avec 70 g de farine auxquelles on ajoute environ 5 5ml d'eau tiède et environ 8 g de beurre ou de margarine.

Cette composition de prémixe peut aussi être incorporée en une proportion allant de 15 à 50% en poids par rapport au poids total de la composition.

Cette composition peut également être utilisée pour préparer des blends en réduisant voire en supprimant la quantité de farine et la dose d'utilisation finale ainsi que pour les mixes des exemples 1 à 6.

### Exemple 8 Composition d'une levure de type « deux en un »

| | **Minimum** | **Maximum** |
|---|---|---|
| - Levure 0,6mm | 99,50 | 98,50 |
| - Améliorant | 0,5 | 1,50 |
| Total | 100,00 | 100,00 |

L'améliorant comprend différents ingrédients, tels que l'acide ascorbique (de 10 à 100 ppm - de préférence de 20 à 50 ppm), l'alpha amylase fongique (de 0,5 à 15 ppm - de préférence de 3 à 10 ppm), des hémicellulases (de 5 à 150 ppm - de préférence de 15 à 80ppm), de la farine de soja (de 0,05 à 0,5% - de préférence de 0,1 à 0,3%), des émulsifiants (Datem de 0,05 à 0,5% - de préférence de 0,1 à 0,3%, et/ou mono et diglycéride de 0,05 à 0,5% - de préférence de 0,1 à 0,3 et/ou SSL de 0,05 à 0,5% - de préférence de 0,1 à 0,3%), de la levure désactivée (de 0,05 à 0,15%).

### Essai comparatif de stabilité

La stabilité du pouvoir ferment, de la levure selon l'invention conservée dans un mixe farine, a été testée selon un test accéléré de laboratoire, et comparée à d'autres levures sèches connues.

Ce test consiste à mélanger la farine avec la levure pour obtenir un mixe, à conserver ce mélange à une température d'environ 30°C, puis à évaluer la perte de force fermentaire par rapport à l'état initial, c'est-à-dire sans conservation. Cette perte de force fermentaire est obtenue par la mesure du dégagement de CO₂ à l'aide d'un fermentomètre, avant conservation et, après 14 jours de conservation du mixe à environ 30°C.

Ce test permet d'étudier le comportement de la levure de l'invention lorsqu'elle est soumise à la fois à un stress hydrique et un stress oxydatif.

La farine est utilisée à environ 14,5% d'humidité.

Les tableaux I et II ci-après rassemblent les résultats obtenus lorsque la levure est respectivement, utilisée en pâte normale (tableau I) et en pâte sucrée (tableau II).

**Tableau I (pâte normale)**

| | **Stabilité** |
|---|---|
| Levure sèche vendue sous la marque commerciale « Aigle du Nord » ou « Saf Instant Rouge » | de 2 à 14% selon l'origine |
| Levure sèche vendue sous la marque commerciale « Yeast in classic » | de 22 à 25% |
| Levure sèche vendue sous la marque commerciale « High Power Plus » | De 1 à 15% |
| Levure de l'invention | de 44 à 49% |

**Tableau II (pâte sucrée)**

| | **Stabilité** |
|---|---|
| Levure sèche vendue sous la marque commerciale « Bruggeman » | de 19 à 29% |
| Levure sèche vendue sous la marque commerciale « Saf Instant Or » | de 39 à 48% |
| Levure sèche vendue sous la marque commerciale « Saf-mix-SPM Gold » | 49% |
| Levure de l'invention | de 61 à 64% |

Les résultats de ces travaux montrent bien que la levure de l'invention présente une meilleure stabilité du pouvoir ferment, en mixe, lorsqu'elle est utilisée en pâte normale et en pâte sucrée.

### Essai comparatif de dispersion

Cet essai consiste à évaluer la dispersion de la levure de l'invention en comparaison à une autre levure sèche connue lorsqu'elle est incorporée dans un aliment à base de farine destiné aux animaux.

Ce test consiste à incorporer un échantillon de levure dans une ration alimentaire puis à mixer l'ensemble.

La ration alimentaire comprend 33% de blé, 33% de farine de soja et 34% de farine de graine de colza. La levure est ajoutée en une quantité de 0,1% par rapport au poids total de la ration alimentaire.

La capacité de dispersion de la levure est obtenue par le calcul du coefficient de variation (CV) selon les références du Centre Technique de l'Alimentation Animale de la société « TECALIMAN ».

Une valeur du coefficient de variation inférieure à 10% indique une bonne dispersion de l'ingrédient ajouté dans la ration alimentaire ; et une valeur de coefficient inférieure à 5% indique une dispersion optimale.

Les résultats sont rassemblés dans le tableau III ci-après.

**Tableau III**

| | **Ajout en %** | **Quantité souhaitée (CFU/g)** | **Coefficient de variation (CV) en %** |
|---|---|---|---|
| Levure sèche témoin (diamètre : 1,8mm) vendue sous marque « Actisaf » | 0,1 | 8.10⁶ | 2,4 |
| Levure selon l'invention (diamètre : 0,5mm) | 0,1 | 7,3.10⁶ | 1,1 |
| Levure selon l'invention (diamètre : 0,6mm) | 0,1 | 9.10⁶ | 1,1 |

Les résultats montrent que la levure selon l'invention présente une excellente dispersion lorsqu'elle est incorporée dans un aliment à base de farine.

## Revendications

1. Procédé de fabrication de levure sèche se présentant sous forme de sphérules entourées d'une couche protectrice périphérique essentiellement constituée de cellules mortes, le procédé de fabrication étant **caractérisé en ce qu'**il consiste à effectuer :
- une déshydratation d'un volume de crème de levure comprenant une saumure à base de chlorure de sodium,
- une granulation de la levure déshydratée pour l'extruder, et
- un séchage de la levure extrudée de manière à obtenir une levure sèche ayant un taux de matière sèche supérieur ou égal à 92% en poids, le séchage étant effectué en discontinu et en l'absence d'additif chimique, et consistant lors d'une première étape, effectuée dans un tambour rotatif, à faire passer un courant d'air chaud dans le volume de levure, puis lors d'une seconde étape, effectuée dans une tour de séchage, à envoyer de l'air chaud sous la partie inférieure du volume de levure.

2. Procédé selon la revendication 1, **caractérisé en ce que** la première étape de séchage dure environ 6 heures, et **en ce que** la seconde étape de séchage dure environ 2 heures.

3. Procédé selon la revendication 1 ou la revendication 2, **caractérisé en ce que** pendant la première étape le courant d'air chaud a une température comprise entre environ 35°C et 38°C, et **en ce que** pendant la seconde étape le courant d'air chaud a une température comprise entre environ 41°C et 60°C.

4. Procédé selon l'une des revendications 2 à 3, **caractérisé en ce que** pendant la première étape le courant d'air chaud a un débit compris entre environ 20 000 et 30 000 m³/h, et **en ce que** pendant la seconde étape le courant d'air chaud a un débit d'environ 2700 m³/h à 4600 m³/h.

5. Procédé selon la revendication 1, **caractérisé en ce que** la déshydratation se fait sous vide d'air.

6. Levure sèche obtenue par le procédé selon l'une des revendications précédentes, **caractérisée en ce qu'**elle présente un diamètre moyen compris entre environ 0,4 et 0,7 mm, **en ce qu'**elle ne comprend pas d'additif chimique, et **en ce qu'**elle présente une durée de reprise d'humidité comprise entre environ 1 et 2 heures.

7. Levure selon la revendication 6, **caractérisée en ce qu'**elle présente un diamètre moyen d'environ 0,55 mm.

8. Composition **caractérisée en ce qu'**elle comprend de la levure selon la revendication 6 ou la revendication 7, au moins un ingrédient choisi parmi le gluten, le sel, les améliorants, les levains, différentes farines de blé ou de seigle et leur mélange, chaque ingrédient ayant, individuellement ou en mélange un taux d'humidité compris entre environ 1 et 16% en poids.

9. Composition selon la revendication 8, **caractérisée en ce que** la levure est comprise en une quantité entre environ 0,40 et 99,9% en poids par rapport au poids total de la composition.

10. Utilisation de la levure selon la revendication 6 ou la revendication 7 dans une composition destinée à préparer une pâte boulangère sucrée ou non.

11. Utilisation de la levure selon la revendication 6 ou la revendication 7 dans une composition alimentaire pour animal.

12. Utilisation de la levure selon la revendication 6 ou la revendication 7 en brasserie et/ou en oenologie.

13. Composition **caractérisée en ce qu'**elle comprend de la levure selon la revendication 6 ou la revendication 7, **en ce qu'**elle se présente sous forme de gélule et/ou de stick et **en ce qu'**elle ne comprend pas d'excipient.

## Patentansprüche

1. Verfahren zur Herstellung einer Trockenhefe, welche in Form von Kügelchen vorliegt, welche mit einer peripheren Schutzschicht umgeben sind, welche im Wesentlichen aus toten Zellen besteht, wobei das Herstellungsverfahren **dadurch gekennzeichnet ist, dass** es besteht aus der Durchführung:
- einer Entwässerung eines Volumens einer Hefecreme umfassend eine Salzlösung auf Grundlage von Natriumchlorid,
- einer Granulierung der entwässerten Hefe, um sie zu extrudieren, und
- einer Trocknung der extrudierten Hefe, um eine Trockenhefe zu erhalten, welche eine Trockenmasse oberhalb oder gleich von 92 Gew.-% aufweist, wobei die Trocknung diskontinuierlich und in Abwesenheit eines chemischen Additivs durchgeführt wird und während des ersten Schritts, welcher in einer rotierenden Trommel durchgeführt wird, aus dem Leiten eines Heißluftstroms in das Hefevolumen, dann während eines zweiten Schritts, welcher in einem Trockenturm durchgeführt wird, aus dem Schicken von heißer Luft unter den unteren Teil des Hefevolumens besteht.

2. Verfahren nach Anspruch 1, **dadurch gekennzeichnet, dass** der erste Trockenschritt etwa 6 Stunden dauert, und dadurch, dass der zweite Trockenschritt etwa 2 Stunden dauert.

3. Verfahren nach Anspruch 1 oder Anspruch 2, **dadurch gekennzeichnet, dass** während des ersten Schritts der Heißluftstrom eine Temperatur umfasst zwischen etwa 35 °C und 38 °C aufweist, und dadurch, dass während des zweiten Schritts der Heißluftstrom eine Temperatur umfasst zwischen etwa 41 °C und 60 °C aufweist.

4. Verfahren nach einem der Ansprüche 2 bis 3, **dadurch gekennzeichnet, dass** während des ersten Schritts der Heißluftstrom einen Durchfluss umfasst zwischen etwa 20 000 und 30 000 m³/h aufweist, und dadurch, dass während des zweiten Schritts der Heißluftstrom einen Durchfluss von etwa 2700 m³/h bis 4600 m³/h aufweist.

5. Verfahren nach Anspruch 1, **dadurch gekennzeichnet, dass** die Entwässerung im Vakuum durchgeführt wird.

6. Trockenhefe erhalten durch das Verfahren nach einem der vorstehenden Ansprüche, **dadurch gekennzeichnet, dass** sie einen mittleren Durchmesser umfasst zwischen etwa 0,4 und 0,7 mm aufweist, und dadurch, dass sie kein chemisches Additiv umfasst, und dadurch, dass sie eine Dauer zur Wiederaufnahme von Feuchtigkeit umfasst zwischen etwa 1 und 2 Stunden aufweist.

7. Trockenhefe nach Anspruch 6, **dadurch gekennzeichnet, dass** sie einen mittleren Durchmesser von etwa 0,55 mm aufweist.

8. Zusammensetzung, **dadurch gekennzeichnet, dass** die Hefe nach Anspruch 6 oder Anspruch 7, wenigstens einen Bestandteil ausgewählt aus Gluten, Salz, Verstärkern, Sauerteig, unterschiedlichen Mehlen von Weizen oder Roggen und ihrer Mischung umfasst, wobei jeder Bestandteil einzeln oder als Gemisch einen Feuchtigkeitsgehalt umfasst zwischen etwa 1 und 16 Gew.-% aufweist.

9. Zusammensetzung nach Anspruch 8, **dadurch gekennzeichnet, dass** die Hefe in einer Menge zwischen etwa 0,40 und 99,9 Gew.-% in Bezug auf das Gesamtgewicht der Zusammensetzung umfasst ist.

10. Verwendung der Hefe nach Anspruch 6 oder Anspruch 7 in einer Zusammensetzung, welche zur Herstellung von gezuckerten oder nicht gezuckerten Teigwaren bestimmt ist.

11. Verwendung der Hefe nach Anspruch 6 oder Anspruch 7 in einer Nahrungszusammensetzung für Tiere.

12. Verwendung der Hefe nach Anspruch 6 oder Anspruch 7 zum Brauen oder/und zur Herstellung von Wein.

13. Zusammensetzung, **dadurch gekennzeichnet, dass** sie die Hefe nach Anspruch 6 oder Anspruch 7 umfasst, und dadurch, dass sie in Form einer Kapsel und/oder eines Stifts vorliegt, und dadurch, dass sie keinen Hilfsstoff umfasst.

## Claims

1. Method for producing dry yeast in the form of spherules surrounded by a protective peripheral layer essentially consisting of dead cells, the production method being **characterized in that** it consists in:
• dehydrating a volume of cream yeast comprising a sodium chloride-based brine,
• granulating the dehydrated yeast in order to extrude it, and
• drying the extruded yeast so as to obtain a dry yeast having a dry matter content greater than or equal to 92% by weight, the drying being carried out batchwise and in the absence of any chemical additive, and consisting, during a first step, carried out in a rotary drum, in passing a stream of hot air through the volume of yeast, and then, during a second step, carried out in a drying tower, in sending hot air under the lower part of the volume of yeast.

2. Method according to Claim 1, **characterized in that** the first drying step lasts approximately 6 hours, and **in that** the second drying step lasts approximately 2 hours.

3. Method according to Claim 1 or Claim 2, **characterized in that**, during the first step, the stream of hot air has a temperature of between approximately 35°C and 38°C, and **in that**, during the second step, the stream of hot air has a temperature of between approximately 41°C and 60°C.

4. Method according to either of Claims 2 and 3, **characterized in that**, during the first step, the stream of hot air has a flow rate of between approximately 20 000 and 30 000 m³/h, and **in that**, during the second step, the stream of hot air has a flow rate of approximately 2700 m³/h to 4600 m³/h.

5. Method according to Claim 1, **characterized in that** the dehydration is carried out under an air vacuum.

6. Dry yeast obtained by means of the method according to one of the preceding claims, **characterized in that** it has an average diameter of between approximately 0.4 and 0.7 mm, **in that** it does not comprise any chemical additive, and **in that** it has a moisture uptake time of between approximately 1 and 2 hours.

7. Yeast according to Claim 6, **characterized in that** it has an average diameter of approximately 0.55 mm.

8. Composition, **characterized in that** it comprises yeast according to Claim 6 or Claim 7, at least one ingredient chosen from gluten, salt, improvers, leavens, various wheat or rye flours, and a mixture thereof, each ingredient having, individually or as a mixture, a moisture content of between approximately 1 and 16% by weight.

9. Composition according to Claim 8, **characterized in that** the yeast is included in an amount of between approximately 0.40 and 99.9% by weight, relative to the total weight of the composition.

10. Use of the yeast according to Claim 6 or Claim 7, in a composition intended for preparing a sweetened or unsweetened baker's dough.

11. Use of the yeast according to Claim 6 or Claim 7, in an animal feed composition.

12. Use of the yeast according to Claim 6 or Claim 7, in brewing and/or in enology.

13. Composition **characterized in that** it comprises yeast according to Claim 6 or Claim 7, **in that** it is in gel and/or stick form, and **in that** it does not comprise any excipient.
